# EUROPÄISCHE PATENTSCHRIFT

(11) **EP 3 771 479 B1**
(45) Veröffentlichungstag und Bekanntmachung des Hinweises auf die Patenterteilung: **25.12.2024**
(21) Anmeldenummer: 19189736.2
(22) Anmeldetag: 02.08.2019
(51) Int. Cl.: A61N 1/39

(54) **THERAPIE BEI ELEKTRODENFEHLER**
TREATMENT IN THE EVENT OF ELECTRODE ERROR
THÉRAPIE LORS D'UN DÉFAUT D'ÉLECTRODE

(43) Veröffentlichungstag der Anmeldung: 03.02.2021
(73) Patentinhaber: BIOTRONIK SE & Co. KG, 12359 Berlin (DE)
(72) Erfinder: Dörr, Thomas, 12437 Berlin (DE); Kucher, Andreas, 16303 Schwedt (DE)
(74) Vertreter: Biotronik Corporate Services SE

(56) Entgegenhaltungen:
- US-A- 5 591 211
- US-B1- 7 031 773
- US-B1- 7 574 259

## Beschreibung

Die Erfindung betrifft ein implantierbares medizinisches Gerät zur Defibrillation des Herzens eines Patienten sowie ein Verfahren zur Steuerung eines derartigen medizinischen Gerätes.

Solche implantierbaren medizinischen Geräte werden auch als implantierbare Kardioverter-Defibrillator (auch kurz ICD für implantable cardioverter-defibrillator) bezeichnet. Ein solches medizinisches Gerät ist dazu ausgebildet, ein Kammerflimmern des Herzens des Patienten zu erfassen und durch Abgabe eines elektrischen Schocks bzw. Strompulses zu therapieren.

Problematisch bei einem implantierbaren medizinischen Gerät mit Defibrillationsfunktion ist der Umstand, dass es aufgrund der vergleichsweise hohen Spannung zur Erzeugung eines Strompulses im Falle eines Isolationsdefektes einer Elektrode zu Hochspannungsüberschlägen kommen kann, der einen sehr hohen Fehlerstrom zur Folge hat. Aktuelle ICD besitzen eine elektronische Kurzschlusssicherung für diesen Fehlerfall, so dass der ICD keine Bauteilschädigung im Kurzschlussfall erfährt und weiter in der Lage ist, seine diagnostischen und therapeutischen Funktionen auszuführen, jedoch mit der Einschränkung, dass die Hochspannungstherapie inneffektiv bleibt.

Die US2010228307 beschreibt in dieser Hinsicht ein Reduzieren der Schockenergie beim ICD wenn Elektrodenfehler erkannt werden, um einen Spannungsüberschlag von der Elektrodenfehlstelle auf das Gerätegehäuse zu verhindern.

Weiterhin ist es im Stand der Technik bekannt, im Falle eines erkannten Kurzschlusses im Schockelektrodenpfad die Schockelektrodenkonfiguration bzw. der Schockpfad für nachfolgende Therapieversuche umzuschalten, sofern eine sogenannte Dual-Coil-Elektrode verwendet wird. Dual-Coil Elektroden verfügen über eine rechtsventrikuläre Schockspule (RV-Coil) und eine zweite Schockspule in der oberen Hohlvene (Superior Vena Cava, SVC-Coil). Verschiedene Schockpfade können zwischen RV-Coil, SVC-Coil und Implantatgehäuse gewählt werden. Siehe hierzu Swerdlow et al, "Implantable Cardiac Defibrillator Lead Failure and Management", JOURNAL OF THE AMERICAN COLLEGE OF CARDIOLOGY, VOL. 67, NO. 11, 2016. Ein Gerät nach der Präambel von Anspruch 1 ist aus US 7 031 773 B1 bekannt.

Das Ausbleiben einer Hochspannungstherapie durch Kurzschlusssicherung ist allerdings unerwünscht, da hierdurch dem Patienten ggf. eine notwendige Therapie nicht bereitgestellt werden kann. Grundsätzlich ist es wünschenswert, dem Patienten auch im oben beschriebenen Fehlerfall eine Hochspannungstherapie / Defibrillation bereitstellen zu können, die insbesondere nicht durch die Verwendung einer spezifischen Schockelektrode eingeschränkt ist.

Der Erfindung liegt daher hiervon ausgehend die Aufgabe zugrunde, ein implantierbares medizinisches Gerät zur Defibrillation des Herzens eines Patienten zu schaffen, das im Falle eines Isolationsdefektes einer Defibrillationselektrode mit Kurzschlusserkennung und Auslösung der Kurzschlusssicherung im Gerät in der Lage ist, dennoch weitere Therapieversuche durchzuführen, die eine in dieser Situation erhöhte Erfolgswahrscheinlichkeit für die Terminierung einer lebensbedrohlichen ventrikulären Tachyarrhythmie aufweisen.

Weiterhin liegt der Erfindung die Aufgabe zugrunde, ein entsprechendes Verfahren zum Steuern einer solchen implantierbaren medizinischen Vorrichtung anzugeben.

Diese Aufgaben werden durch ein implantierbares medizinisches Gerät mit den Merkmalen des Anspruchs 1 sowie durch ein Verfahren mit den Merkmalen des Anspruchs 7 gelöst.

Bevorzugte Ausführungsformen dieser Erfindungsaspekte sind in den entsprechenden Unteransprüchen angegeben und werden nachfolgend beschrieben.

Gemäß Anspruch 1 wird ein implantierbares medizinisches Gerät offenbart, das zur Defibrillation des Herzens eines Patienten ausgebildet ist und weiterhin aufweist:
- einen Energiespeicher, der zum Bereitstellen einer Spannung zumindest zwei Kondensatoren aufweist,
- zumindest eine Elektrode zum Abgeben eines elektrischen Strompulses mittels der Spannung, und
- eine Kurzschlusssicherung, die dazu ausgebildet ist, im Falle eines Kurzschlusses der mindestens einen Elektrode auszulösen und eine Abgabe eines elektrischen Strompulses abzubrechen oder zu verhindern, wobei das implantierbare medizinische Gerät dazu ausgebildet ist, im Falle des Kurzschlusses die Abgabe eines weiteren Strompulses, insbesondere mittels einer reduzierten Spannung, aus einer Parallelschaltung der mindestens zwei Kondensatoren zu veranlassen.

Reduzierte Spannung bedeutet hierbei, dass der weitere Strompuls mit einer Spannung abgegeben wird, die gegenüber der Spannung des abgebrochenen bzw. verhinderten Strompulses reduziert ist.

Die Erfindung ermöglicht somit die Abgabe eines angepassten Hochvolttherapieversuches (Defibrillation) für den Fall eines vorliegenden Elektrodendefektes im Hochvolttherapiepfad, indem im Falle eines erkannten Elektrodenfehlers die Therapiespannung vorzugsweise derart reduziert wird, dass in dieser Situation eine höhere Therapieerfolgswahrscheinlichkeit resultiert.

Die erfindungsgemäße Lösung hat den Vorteil, dass die Therapie- bzw. Schockspannung im Vergleich zu der eines normalen Defibrillationsschocks reduziert werden kann, jedoch die verabreichte Schockenergie im Wesentlichen gleichbleibt.

Die Erfindung kann insbesondere den Mechanismus eines typischen Kurzschlusses im Defibrillationspfad des Gerätes ausnutzen: In der Regel liegt bei einem Isolationsdefekt der zum Schocken verwendeten Elektrode kein rein metallischer Kontakt zwischen den hochspannungsführenden Leitern oder Bauteilen vor, sondern es kommt im Moment der Hochspannungsabgabe zu einem Spannungsüberschlag bzw. Lichtbogen. Die Wahrscheinlichkeit eines solchen Überschlags steigt mit der angewendeten maximalen Spannung und kann daher reduziert werden, wenn in nachfolgenden Therapieversuchen die maximale Spannung reduziert wird, dies jedoch nur in dem Maße, dass weiterhin ein hinreichender Therapieerfolg möglich ist.

Weiterhin ist gemäß einer bevorzugten Ausführungsform des erfindungsgemäßen implantierbaren medizinischen Geräts vorgesehen, dass das implantierbare medizinische Gerät eine Erkennungsvorrichtung aufweist, die dazu ausgebildet ist, das Auslösen der Kurzschlusssicherung zu erkennen und einer Steuereinheit des implantierbaren medizinischen Geräts zu signalisieren, wobei die Steuereinheit dazu ausgebildet ist, für den Fall einer erkannten Auslösung der Kurzschlusssicherung die Abgabe des weiteren Strompulses mit der vorzugsweise reduzierten Spannung abzugeben.

Weiterhin ist gemäß einer Ausführungsform der Erfindung vorgesehen, dass die Steuereinheit dazu ausgebildet ist, im Falle einer erkannten Auslösung der Kurzschlusssicherung eine Umschaltung einer Reihenschaltung von Kondensatoren in die Parallelschaltung der mindestens zwei Kondensatoren zu veranlassen. Hierbei kann z.B. eine an den beiden in Reihe geschalteten Kondensatoren anliegende Gesamtspannung reduziert werden auf eine geringere Spannung die an den nunmehr parallel geschalteten Kondensatoren anliegt.

Weiterhin ist gemäß einer bevorzugten Ausführungsform des erfindungsgemäßen implantierbaren medizinischen Geräts vorgesehen, dass die mindestens eine Elektrode des implantierbaren medizinische Geräts dazu ausgebildet ist, über eine Vene in eine Herzkammer des Patienten eingeführt zu werden (d.h. das medizinische Gerät ist ein sogenannter transvenöser Kardioverter-Defibrillator).

Weiterhin ist gemäß einer bevorzugten Ausführungsform des erfindungsgemäßen implantierbaren medizinischen Geräts vorgesehen, dass die mindestens eine Elektrode des implantierbaren medizinischen Geräts dazu ausgebildet ist, subkutan implantiert zu werden, und zwar insbesondere oberhalb des Brustbeins des Patienten.

Weiterhin ist gemäß einer bevorzugten Ausführungsform des erfindungsgemäßen implantierbaren medizinischen Geräts vorgesehen, dass die mindestens eine Elektrode des implantierbaren medizinischen Geräts dazu ausgebildet ist, substernal (d.h. unter dem Brustbein des Patienten) implantiert zu werden.

Das implantierbare medizinische Gerät weist vorzugsweise ein Gehäuse zur Aufnahme der mindestens zwei Kondensatoren bzw. des Energiespeichers sowie insbesondere der weiteren Komponenten auf (z.B. Kurzschlusssicherung und/oder Steuereinheit und/oder Erkennungsvorrichtung). Die mindestens eine Elektrode ist mit dem Gehäuse verbunden.

Bei einer subkutanen oder transvenösen Elektrode kann das Gehäuse z.B. dazu ausgebildet sein, in der Brust des Patienten implantiert zu werden (z.B. links seitlich des Brustkorbes, unterhalb der Achsel). Auch bei einer substernalen Elektrode kann das Gehäuse in der Brust des Patienten implantiert werden.

Ein weiterer Aspekt der Erfindung betrifft ein Verfahren zum Steuern eines implantierbaren medizinischen Geräts (insbesondere eines erfindungsgemäßen implantierbaren medizinischen Geräts), aufweisend die Schritte:
- Bereitstellen einer Spannung mittels zumindest zweier Kondensatoren des implantierbaren medizinischen Geräts zum Abgeben eines Strompulses, und
- Abbrechen oder Verhindern einer Abgabe des elektrischen Strompulses im Falle der Erkennung eines Kurzschlusses einer Elektrode des implantierbaren medizinischen Geräts, sowie
- Abgabe eines weiteren Strompulses aus einer Parallelschaltung der mindestens zwei Kondensatoren.

Gemäß einer bevorzugten Ausführungsform des erfindungsgemäßen Verfahrens ist vorgesehen, dass eine Kurzschlusssicherung des implantierbaren medizinischen Geräts im Falle eines Kurzschlusses einer Elektrode ausgelöst wird und damit eine Abgabe eines elektrischen Strompulses mittels des implantierbaren medizinischen Geräts abgebrochen oder verhindert wird, wobei insbesondere im Falle eines Kurzschlusses die Abgabe des weiteren Strompulses aus der Parallelschaltung der mindestens zwei Kondensatoren erfolgt.

Weiterhin ist gemäß einer bevorzugten Ausführungsform des erfindungsgemäßen Verfahrens vorgesehen, dass das Auslösen der Kurzschlusssicherung mittels einer Erkennungsvorrichtung automatisch erkannt wird, wobei die Erkennungsvorrichtung einer Steuereinheit des implantierbaren medizinischen Geräts das Auslösen der Kurzschlusssicherung signalisiert, und wobei die Steuereinheit im Falle einer erkannten Auslösung der Kurzschlusssicherung die Abgabe des weiteren Strompulses veranlasst.

Gemäß einer bevorzugten Ausführungsform des erfindungsgemäßen Verfahrens ist vorgesehen, dass Falle eines Kurzschlusses eine Umschaltung einer Reihenschaltung von Kondensatoren in die Parallelschaltung der mindestens zwei Kondensatoren durch das implantierbare medizinische Gerät automatisch veranlasst wird (z. B. durch die Steuereinheit) und sodann die Abgabe des weiteren Strompulses veranlasst wird.

Im Folgenden sollen Ausführungsformen der Erfindung sowie weitere Merkmale und Vorteile der Erfindung anhand der Figuren erläutert werden. Es zeigen:
- Fig. 1: eine schematische Darstellung eines typischen Fehlermechanismus des implantierbaren medizinischen Geräts, der zu einem Kurzschluss einer Elektrode des Geräts führen kann;
- Fig. 2: eine Reduktion der Spannung des medizinischen Geräts zur Erzeugung eines elektrischen Strompulses für die Defibrillation;
- Fig. 3: eine schematische Darstellung der Umschaltung zwischen einer Reihenschaltung der Kondensatoren des implantierbaren medizinischen Geräts und einer Parallelschaltung der Kondensatoren zur Reduktion der besagten Spannung; und
- Fig. 4: eine weitere Möglichkeit zur Reduktion der Spannung für die Defibrillation.

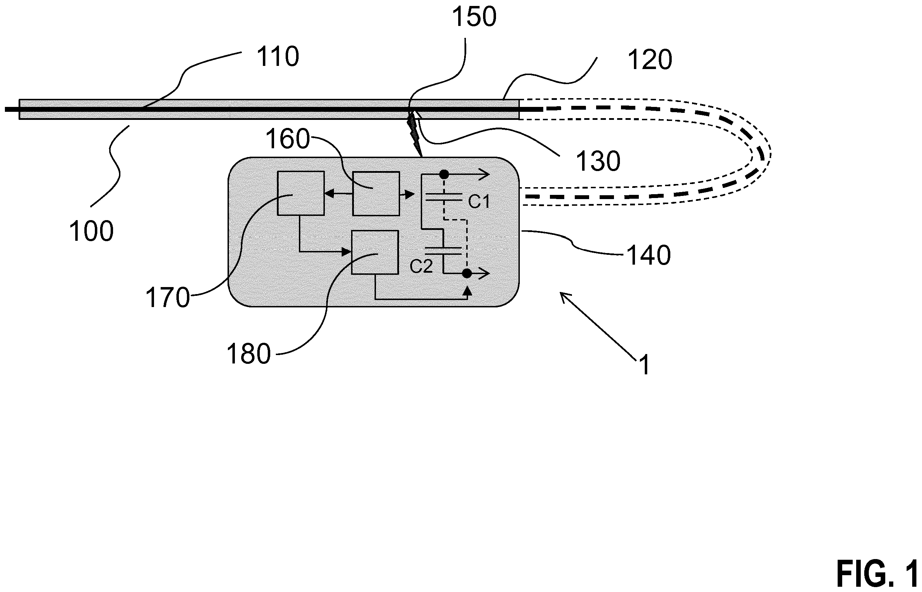

In der Figur 1 ist ein typischer Fehlermechanismus eines Isolationsdefekts bei Defibrillationsschockabgabe (d.h. Abgabe eines elektrischen Strompulses) durch ein erfindungsgemäßes implantierbares medizinisches Gerät 1 illustriert. Die mindestens eine Elektrode 100 ist als Elektrodenleitung 100 ausgeführt und weist insbesondere einen elektrischen Leiter 110 und einen diesen umgebenden Isolator 120 auf, wobei im Fehlerfall dieser Isolator 120 einen Isolationsdefekt 130 aufweist. Die Gegenelektrode für die Defibrillation ist in diesem Fall ein Gehäuse 140 des implantierbaren medizinischen Geräts 1 bzw. Defibrillators 1.

Da sich in dieser Anordnung der elektrische Leiter 110 und das Gehäuse 140 auch im Bereich des Isolationsdefektes 130 typischerweise nicht berühren können, ist eine Erkennung dieses Isolationsdefektes mittels eine Niederspannungsmessung nicht erkennbar, d.h. die sog. schmerzlose Schockimpedanzmessung zeigt hier keine Auffälligkeit.

Wird nun aber eine Hochspannungstherapie über die Elektrode 100 und Gegenelektrode 140 abgegeben, dann kann im Bereich des Isolationsdefektes ein Hochspannungsüberschlag 150 stattfinden, der einen sehr hohen Fehlerstrom zur Folge hat. Das implantierbare medizinische Gerät 1 weist daher eine elektronische Kurzschlusssicherung 160 für diesen Fehlerfall auf, so dass das Gerät 1 keine Bauteilschädigung im Kurzschlussfall erfährt und weiter in der Lage ist, seine diagnostischen und therapeutischen Funktionen auszuführen, jedoch mit der Einschränkung, dass die Hochspannungstherapie inneffektiv bleibt.

Die Kurzschlusssicherung 160 ist dazu ausgebildet, im Falle eines Kurzschlusses der mindestens einen Elektrode 100 auszulösen und eine Abgabe eines elektrischen Strompulses durch das implantierbare medizinische Gerät 1 abzubrechen oder zu verhindern. Weiterhin weist das implantierbare medizinische Gerät 1 bevorzugt eine Erkennungsvorrichtung 170 auf (vgl. Fig. 1), die dazu ausgebildet ist, das Auslösen der Kurzschlusssicherung 160 zu erkennen und einer Steuereinheit 180 des implantierbaren medizinischen Geräts 1 zu signalisieren, wobei die Steuereinheit 180 dazu ausgebildet ist, für den Fall einer erkannten Auslösung der Kurzschlusssicherung 160 einen weiteren elektrischen Strompuls aus einer Parallelschaltung zumindest zweier Kondensatoren C1, C2 zu veranlassen. Gemäß einer Ausführungsform der Erfindung ist die Steuereinheit 180 bzw. das medizinische Gerät 1 dazu konfiguriert, eine Umschaltung einer vorherigen Reihenschaltung der Kondensatoren C1, C2 in eine Parallelschaltung vorzunehmen (gestrichelte Linie zwischen C1 und C2 in Fig. 1), derart, dass die Abgabe des weiteren Strompulses aus der Parallelschaltung mit einer Spannung erfolgt, die gegenüber der zuvor bei dem abgebrochenen oder bei dem verhinderten Strompuls vorgesehenen Spannung der Reihenschaltung der Kondensatoren reduziert ist.

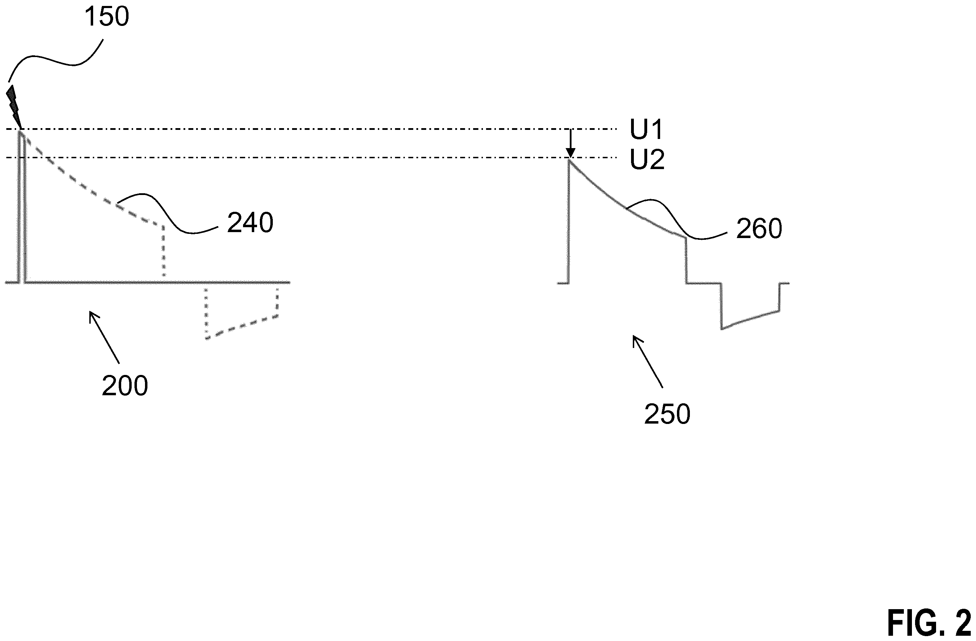

Figur 2 zeigt eine schematische Darstellung der erfindungsgemäßen Reduktion der Spannung bei der Schocktherapieabgabe im Falle eines Isolationsdefektes. Der erste abgegebene Defibrillationsschock bzw. elektrischer Strompuls 200 wird mit der programmierten bzw. maximalen Spannung U1 abgegeben, was einen Hochspanungsüberschlag 150 im Bereich des Isolationsdefektes 130 verursacht und daraufhin die Schockabgabe durch die elektronische Kurzschlusssicherung 160 abgebrochen wird. Die abzugebende Schockenergie 240 wird damit nicht für die Defibrillation wirksam.

Erfindungsgemäß wird in diesem Implementierungsbeispiel nach Erkennung der Auslösung der Kurzschlusssicherung 160 beim nächsten Defibrillationsversuch 250 die maximale Spannung auf einen reduzierten Wert U2 eingestellt (insbesondere durch die oben beschriebene Umschaltung der Kondensatoren C1 und C2), so dass zum einen die Wahrscheinlichkeit eines erneuten Hochspannungsüberschlags reduziert wird, aber noch eine hinreichende Schockenergie 260 für einen wahrscheinlichen Therapieerfolg abgegeben werden kann. Erfolgt in diesem Fall kein erneuter Hochspannungsüberschlag, wird die vorgesehene Defibrillationsenergie abgegeben und es kann damit die Tachyarrhythmie terminiert werden.

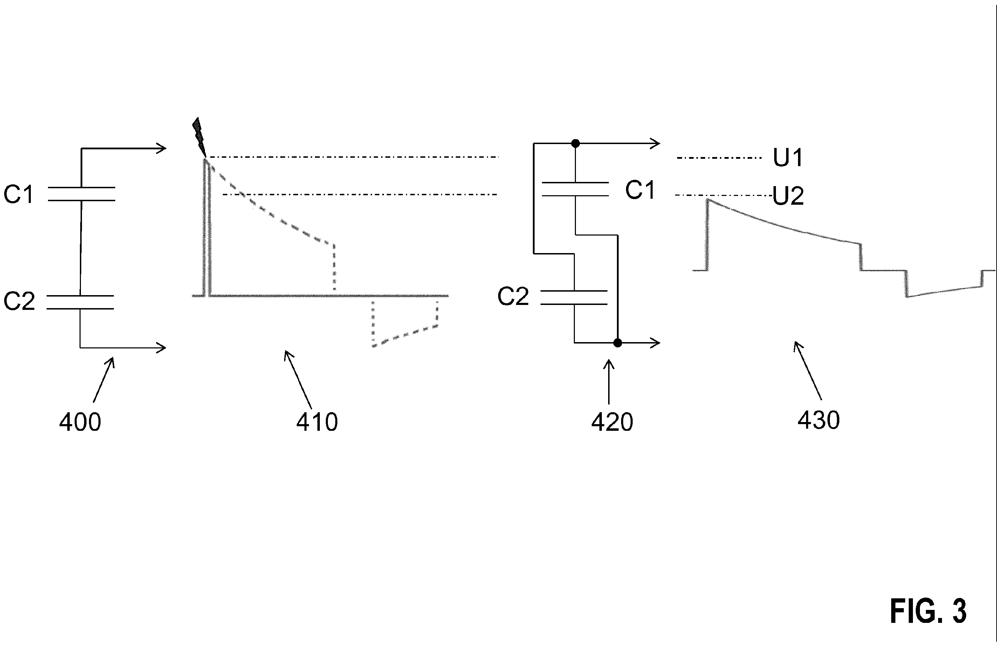

Figur 3 verdeutlicht in schematische Weise die oben beschriebene Umschaltung der Kondensatoren C1, C2. Hier wird zunächst der Defibrillationsschock 410 bzw. elektrische Strompuls aus einer Reihenschaltung 400 der zwei Kondensatoren C1, C2 abgegeben, was im Fehlerfall zu einer Auslösung der Kurzschlusssicherung 160 führt (siehe oben). Der Folgeschock wird nun aus der Parallelschaltung 420 beider Kondensatoren C1, C2 abgegeben, was den Vorteil bietet, dass die Schockspitzenspannung z.B. halbiert wird und gleichzeitig im Wesentlichen die gesamte maximale Schockenergie abgegeben werden kann.

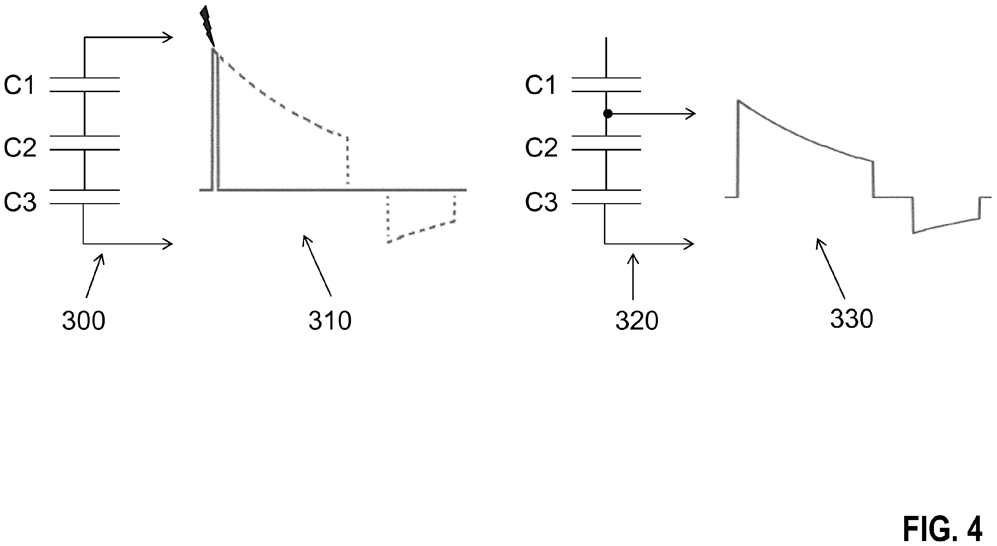

In der Figur 4 ist eine weitere Möglichkeit zur Reduktion der Spannung dargestellt, um eine Defibrillationsschockabgabe mit deutlich verminderter Spitzenspannung und damit Reduktion der Wahrscheinlichkeit eines Hochspannungsüberschlages zu realisieren.

Der erste Schock 310 bzw. elektrische Strompuls zur Defibrillation wird hier aus einer Reihenschaltung 300 von drei Kondensatoren C1, C2, C3 abgegeben, wobei dieser durch einen Isolationsdefekt zu einer Auslösung der Kurzschlusssicherung 160 führt.

Im Folgeschock bzw. Strompuls 330 wird nun mittels elektronischer Schalterelemente (hier nicht dargestellt) der Abgriff für die Schockabgabe nur in einer Reihenschaltung 320 der Kondensatoren C2, C3 eingestellt und damit die Spitzenspannung des Schocks 330 um 1/3 reduziert. In dieser Anordnung erhöht sich die bei der Schockabgabe resultierende Kapazität und damit wird die Dauer der Schockphasen verlängert, was bei geeigneter Dimensionierung der Kapazitäten zu einer höheren Therapieeffizienz bei gleichzeitig geringerer Schockspitzenspannung führen kann.

Die Erfindung erhöht mit Vorteil die Wahrscheinlichkeit, dass im Falle eines Elektrodendefektes eines implantierbaren medizinischen Geräts / Defibrillators eine lebensrettende Therapie dennoch abgegeben werden kann.

## Patentansprüche

1. Implantierbares medizinisches Gerät (1) zur Defibrillation des Herzens eines Patienten, mit:
- einem Energiespeicher zum Bereitstellen einer Spannung,
- zumindest einer Elektrode (100), wobei das implantierbare medizinische Gerät zum Abgeben eines elektrischen Strompulses durch besagte Elektrode für einen Defibrillationsschock mittels der Spannung ausgebildet ist, **gekennzeichnet durch**
- eine Kurzschlusssicherung (160), die dazu ausgebildet ist, im Falle eines Kurzschlusses der mindestens einen Elektrode (100) auszulösen und eine Abgabe eines elektrischen Strompulses für einen Defibrillationsschock durch besagte Elektrode abzubrechen oder zu verhindern, wobei der Energiespeicher zum Bereitstellen der Spannung zumindest zwei Kondensatoren (C1, C2) aufweist,
wobei das implantierbare medizinische Gerät (1) dazu ausgebildet ist, im Falle des Kurzschlusses die Abgabe eines weiteren Strompulses für einen Defibrillationsschock mit reduzierter Spannung aus einer Parallelschaltung der mindestens zwei Kondensatoren (C1, C2) durch besagte Elektrode zu veranlassen.

2. Implantierbares medizinisches Gerät nach Anspruch 1, **dadurch gekennzeichnet, dass** das implantierbare medizinische Gerät (1) eine Erkennungsvorrichtung (170) aufweist, die dazu ausgebildet ist, das Auslösen der Kurzschlusssicherung (160) zu erkennen und einer Steuereinheit (180) des implantierbaren medizinischen Geräts (1) zu signalisieren, wobei die Steuereinheit (180) dazu ausgebildet ist, für den Fall einer erkannten Auslösung der Kurzschlusssicherung (160) die Abgabe des weiteren Strompulses zu veranlassen.

3. Implantierbares medizinisches Gerät nach Anspruch 2, **dadurch gekennzeichnet, dass** die Steuereinheit (180) dazu ausgebildet ist, im Falle einer erkannten Auslösung der Kurzschlusssicherung (160) eine Umschaltung einer Reihenschaltung (400) von Kondensatoren (C1, C2) in die Parallelschaltung (420) der mindestens zwei Kondensatoren (C1, C2) zu veranlassen.

4. Implantierbares medizinisches Gerät nach einem der vorhergehenden Ansprüche, **dadurch gekennzeichnet, dass** die mindestens eine Elektrode (100) des implantierbaren medizinischen Geräts (1) dazu ausgebildet ist, über eine Vene in eine Herzkammer des Patienten eingeführt zu werden.

5. Implantierbares medizinisches Gerät nach einem der vorhergehenden Ansprüche, **dadurch gekennzeichnet, dass** die mindestens eine Elektrode (100) des implantierbaren medizinischen Geräts (1) dazu ausgebildet ist, subkutan implantiert zu werden.

6. Implantierbares medizinisches Gerät nach einem der vorhergehenden Ansprüche, **dadurch gekennzeichnet, dass** die mindestens eine Elektrode (100) des implantierbaren medizinischen Geräts (1) dazu ausgebildet ist, substernal implantiert zu werden.

## Claims

1. An implantable medical device (1) for defibrillating the heart of patient, comprising:
- an energy storage device for providing a voltage,
- at least one electrode (100), wherein the implantable medical device is configured to deliver an electrical current pulse through said electrode for a defibrillation shock by means of the voltage, **characterized by**
- a short-circuit protection (160) which is configured to be triggered in the event of a short-circuit of the at least one electrode (100) and to interrupt or prevent delivery of an electrical current pulse for a defibrillation shock by said electrode, wherein the energy storage device for providing the voltage comprises at least two capacitors (Cl, C2),
wherein, in the event of a short-circuit, the implantable medical device (1) is configured to cause the delivery of a further current pulse for a defibrillation shock with reduced voltage from a parallel circuit of the at least two capacitors (Cl, C2) by said electrode.

2. The implantable medical device according to claim 1, **characterized in that** the implantable medical device (1) has a detection device (170) which is configured to detect the triggering of the short-circuit protection (160) and to signal it to a control unit (180) of the implantable medical device (1), wherein the control unit (180) is configured to initiate the delivery of the further current pulse in the event of a detected triggering of the short-circuit protection (160).

3. The implantable medical device according to claim 2, **characterized in that** the control unit (180) is configured, in the event of a detected triggering of the short-circuit protection (160), to switch a series circuit (400) of capacitors (Cl, C2) to the parallel circuit (420) of the at least two capacitors (Cl, C2).

4. The implantable medical device according to any one of the preceding claims, **characterized in that** the at least one electrode (100) of the implantable medical device (1) is configured to be introduced into a heart chamber of the patient via a vein.

5. The implantable medical device according to any one of the preceding claims, **characterized in that** the at least one electrode (100) of the implantable medical device (1) is configured to be implanted subcutaneously.

6. The implantable medical device according to any one of the preceding claims, **characterized in that** the at least one electrode (100) of the implantable medical device (1) is configured to be implanted substemally.

## Revendications

1. Dispositif médical implantable (1) permettant la défibrillation du coeur d'un patient, pourvu
- d'un dispositif de stockage d'énergie permettant la fourniture d'une tension,
- d'au moins une électrode (100), où le dispositif médical implantable qui est conçu pour la délivrance d'une impulsion de courant électrique par ladite électrode pour un choc de défibrillation au moyen de la tension, **caractérisé par**
- un fusible contre les courts-circuits (160) qui est conçu, dans le cas d'un court-circuit de l'au moins une électrode (100), pour se déclencher et interrompre ou empêcher une délivrance d'une impulsion de courant électrique pour un choc de défibrillation par ladite électrode, où le dispositif de stockage d'énergie, pour la fourniture de la tension, présente au moins deux condensateurs (C1, C2),
où le dispositif médical implantable (1) est conçu, dans le cas du court-circuit, pour organiser la délivrance d'une nouvelle impulsion de courant électrique pour un choc de défibrillation avec une tension réduite à partir d'un circuit parallèle des au moins deux condensateurs (C1, C2) par ladite électrode.

2. Dispositif médical implantable selon la revendication 1, **caractérisé en ce que** le dispositif médical implantable (1) présente un dispositif de reconnaissance (170) qui est conçu pour reconnaître le déclenchement du fusible contre les courts-circuits (160) et signaler à une unité de commande (180) du dispositif médical implantable (1), où l'unité de commande (180) est conçue pour, dans le cas d'un déclenchement reconnu du fusible contre les courts-circuits (160) organiser la délivrance de la nouvelle impulsion de courant.

3. Dispositif médical implantable selon la revendication 2, **caractérisé en ce que** l'unité de commande (180) est conçue, dans le cas d'un déclenchement reconnu du fusible contre les courts-circuits (160), pour organiser une commutation d'un circuit en série (400) de condensateurs (C1, C2) dans le circuit parallèle (420) des au moins deux condensateurs (C1, C2).

4. Dispositif médical implantable selon l'une des revendications précédentes, **caractérisé en ce que** l'au moins une électrode (100) du dispositif médical implantable (1) est conçue pour pouvoir être transférée dans une chambre cardiaque du patient par le biais d'une veine.]

5. Dispositif médical implantable selon l'une des revendications précédentes, , **caractérisé en ce que** l'au moins une électrode (100) du dispositif médical implantable (1) est conçue pour pouvoir être implantée de manière sous-cutanée.

6. Dispositif médical implantable selon l'une des revendications précédentes, **caractérisé en ce que** l'au moins une électrode (100) du dispositif médical implantable (1)est conçue pour pouvoir être implantée de manière substernale.
